# FASCICULE DE BREVET EUROPEEN

(11) **EP 1 501 528 B1**
(45) Date de publication et mention de la délivrance du brevet: **09.01.2013**
(21) Numéro de dépôt: 03746009.4
(22) Date de dépôt: 27.03.2003
(51) Int. Cl.: A61K 36/185, A61K 31/17, A61P 39/06

(54) **PROCEDE D'EXTRACTION DE NN' DIBENZYLTHIOUREE A PARTIR DE LA RACINE D'UNE PLANTE DE LA FAMILLE DES PENTADIPLANDRACEES**
VERFAHREN ZUR EXTRAKTION VON N,N'-DIBENZYLTHIOHARNSTOFF AUS DEN WURZELN EINER PFLANZE DER PENTADIPLANDRACEAE-FAMILIE
METHOD FOR EXTRACTING N,N'-DIBENZYLTHIOUREA FROM THE ROOT OF A PLANT OF THE PENTADIPLANDRACEAE FAMILY

(30) Priorité: 05.04.2002 CM 20020098
(43) Date de publication de la demande: 02.02.2005
(73) Titulaire: Wandji, Thomas, 93160 Noisy Le Grand (FR)
(72) Inventeur: Wandji, Thomas, 93160 Noisy Le Grand (FR)
(86) Numéro de dépôt international: PCT/OA2003/000001
(87) Numéro de publication internationale: WO 2003/084557

(56) Documents cités:
- WO-A-01/85681
- FR-A- 2 157 779
- FR-A- 2 693 106
- GB-A- 887 174
- US-A- 3 391 107
- BATTERA-ARELLANO D ET AL: "ANTIOXIDANT ACTIVITY OF BRAZILIAN PLANT EXTRACT" SOFW-JOURNAL SEIFEN, OELE, FETTE, WACHSE, VERLAG FUR CHEMISCHE INDUSTRIE, H. ZIOLKOWSKY K.G. AUGSBURG, DE, vol. 116, no. 2, 1 février 1990 (1990-02-01), pages 56-58, XP000136050 ISSN: 0942-7694
- MIGIRAB EL S ET AL: "ISOTHIOCYANATES, THIOUREES ET THIOCARBAMATES ISOLES DE PENTADIPLANDRA BRAZZEANA" PHYTOCHEMISTRY, PERGAMON PRESS, GB, vol. 16, 1977, pages 1719-1721, XP009014830 ISSN: 0031-9422

## Description

La présente invention a pour objet un procédé d'extraction d'un compose, NN' DIBENZYLTHIOUREE de la racine d'une plante de la famille des PENTADIPLANDRACEES, genre PENTADIPLANDRA BRAZZEANA, variété BRAZZEANA

Cette plante est une liane que l'on rencontre dans les forêts d'Afrique centrale. Elle est bien connue des botanistes et des éthnobotanistes, sa racine est utilisée comme condiment par certaines populations d'Afrique centrale.

Le document FR 2157779 décrit un procédé d'extraction de trois alcaloïdes de la racine de DiFeu.

Les études phytochimiques ont été faites sur cette plante, mais aucune n'a isolé la NN' DIBENZYLTHIOUREE et mis en évidence son pouvoir antioxydant biologique et en montrer le mécanisme d'action.

Le procédé d'extraction lié à la présente invention permet d'isoler la NN' DIBENZYLTHIOUREE. 1kg de racines broyées et séchées sont mises dans un flacon de couleur jaune de 2 litres. On y ajoute 1 litre d'alcool éthylique à 95° comme solvant.

Ce flacon bouché est monté sur un agitateur mécanique, on laisse l'appareil agiter le flacon pendant 4 heures pour bien extraire le composé. On arrête l'appareil et laisse le flacon au repos pendant 1 heure. On filtre et recueille le filtrat de couleur jaune citron. On chasse l'alcool par distillation sous vide à environ 15 mm HG dans un évaporateur rotatif à bain-marie Thermostat réglé à 40°. Il faut éviter d'aboutir à un résidu sec.

Pour obtenir la NN' DIBENZYLTHIOUREE, on met la solution alcoolique concentrée dans une ampoule à décanter dans laquelle on introduit 250 ml de TETRACHLORURE de Carbone, on agite à la main pendant 1 heure. Au repos il apparaît 2 phases dans l'ampoule à décanter.

La phase hydroalcoolique contient la NN' DIBENZYLTHIOUREE, qui n'est pas soluble dans le TETRACHLORURE de Carbone

Nous avons observé que quelques ml de cette solution évaporée à sec donne un résidu noirâtre et collant en présence de l'oxygène de l'air. L'étude structurale de ce résidu montre qu'il s'agit d'un polymère de la NN' DIBENZYLTHIOUREE sans effets pharmacologiques.

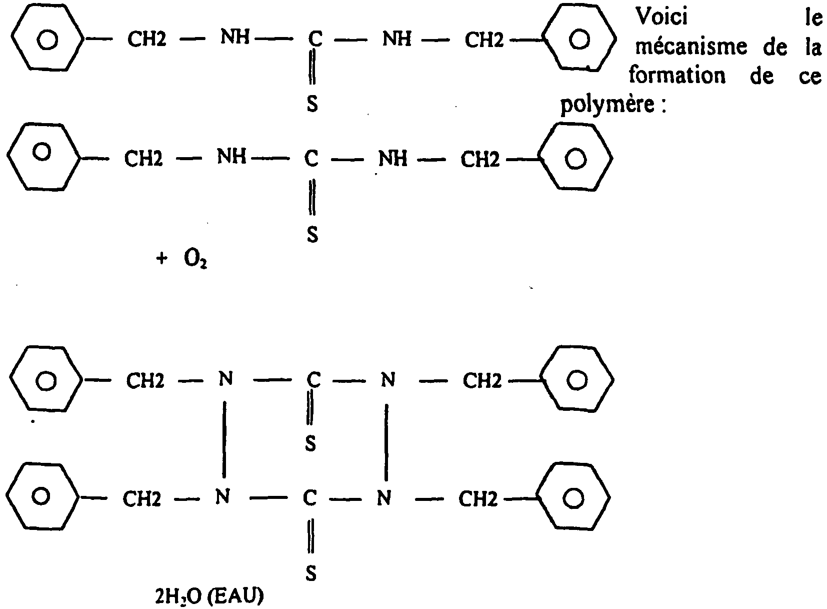

Il ressort de cette réaction la formation d'un polymère à partir de 2 molécules de NN' DIBENZYL THIOUREE avec perte de 4 atomes d'hydrogène qui au contact de l'air donnent 2 molécules d'eau. Nous avons ainsi mis en évidence un anti-oxydant biologique dans la racine du PENTADIPLANDRA BRAZZEANA variété BRAZZEANA c'est la NN' DIBENZYL THIOUREE en solution après l'analyse structurale.

Pour que cette substance soit utilisée comme médicament chez l'homme ou chez l'animal, nous avons démontré invitro l'action de cet anti-oxydant biologique sur le radical hydroxyl (OH°) dont l'agressivité altère l'état et le fonctionnement de nombreuses cellules de l'organisme humain et animal.

Nous avons utilisé la méthode de fentom.

Cette méthode est basée sur la dégradation du desoxyribose par les radicaux libres générés par le système xanthine / xanthine oxydase. Cette dégradation conduit à la formation de malondialdehyde qui est dosé par colorimétrie.

Dans cette technique, l'effet piégeur des radicaux libres hydroxyls se traduit par une diminution de la formation de malondiadehyde.

La NN' DIBENZYL THIOUREE en solution aux concentrations de 20% ; 10% ; 5% diminue la production du malondiadehyde de 65% , de 59 % et 51% respectivement par rapport au témoin ne contenant aucun piégeur, des radicaux libres hydroxyls. Cet anti-oxydant biologique en solution alcoolique agit de la manière suivante dans l'organisme vivant.

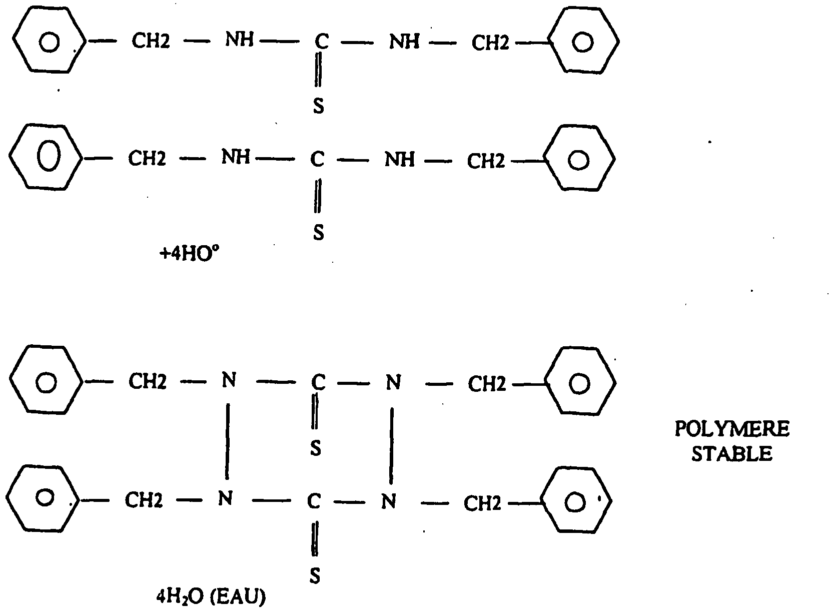

Les produits de cette réaction sont stables et sont éliminés par voie urinaire.

## Revendications

1. Procédé d'extraction de NN' dibenzylthiourée à partir de racines d'une plante de la famille des pentadiplandracées, genre pentadiplandra brazzeana, variété brazzeana, **caractérisé en ce qu'**il comprend les étapes suivantes :
1) Mettre 1kg de racines broyées et séchées dans un flacon de couleur jaune de 2 litres.
2) Addition de 1l d'alcool éthylique à 95°, puis agitation du mélange pendant 4 heures.
3) Repos pendant 1 heure du mélange obtenu à l'étape 2.
4) Filtration et recueil du filtrat.
5) Distillation sous vide à environ 15 mm HG à 40°c jusqu'à obtention d'une solution hylroalcoolique brute concentrée en évitant d'obtenir un résidu sec.
6) Addition de 250ml de Tétrachlorure de carbone à la solution hydroalcoolique obtenue à l'étape 5.
7) Agitation pendant une heure.
8) Décantation et recueil de la phase hydroalcoolique finale contenant la NN' de benzylthiourée non soluble dans le tétrachlorure de carbone.

2. Procédé selon la revendication 1, **caractérisée en ce que** le procédé se fait sans précipitation du produit et sans évaporation totale du solvant afin d'éviter le contact avec l'oxygène de l'air.

3. Procédé selon la revendication 1, **caractérisée en ce que** la phase hydroalcoolique contient la NN' dibenzylthiourée comme unique composé extrait de la racine de pentadiplandra brazzeana, variété brazzeana, non soluble dans le Tétrachlorure de carbone.

4. Procédé selon les revendications 1, 2 et 3, **caractérisées en ce que** la NN' dibenzylthiourée obtenue par ce procédé, est un antioxydant biologique d'origine végétale.

5. Procédé selon les revendications 1, 2, 3 et 4, **caractérisées en ce que** les H du groupement -NH-, qui constitue les sites actifs du composé sont conservés.

## Claims

1. A method of extracting NN' dibenxylthiourea from the roots of a plant of the pentadiplandracea family, Pentadiplandra brazzeana genus, variety brazzeana, **characterized in that** it comprises the following steps;
1). Put 1 kg of ground and dried roots in a yellow bottle of 2 liters.
2). Addition of 1 liter of ethyl alcohol at 95 °, then stirring the mixture for 4 hours.
3). Stand for 1 hour the mixture obtained in step 2.
4). Filtration and collection of the filtrate.
5). Vacuum distillation was about 15 mm Hg at 40°c until a solution is obtained by avoiding crude hydro alcoholic obtains a dry residue.
6). Addition of 250ml of Tetrachloride in hydro-alcoholic solution obtained in step 5.
7). Agitation for 1 hour
8). Settling and collection of the aqueous-alcoholic phase containing the final NN' benzylthiourea not soluble in carbon tetrachloride.

2. The method of claim 1, **characterized in that** the method is done without precipitation of the product and total evaporation of the solvent in order to avoid contact with atmospheric oxygen.

3. The method of claim 1, **characterized in that** the phase contains the NN 'dibenzylthiourea as single root extract consists of Pentadiplandra brazzeanna, brazeanna variety is not soluble in carbon tetrachloride.

4. Process according to claims 1, 2 and 3, **characterized in that** the NN 'dibenzylthiourea obtained by this process, is a biological antioxidant of plant origin.

5. A method according to claims 1, 2, 3 and 4, **characterized in that** the H of -NH- group, is that the active sites of the compound are kept.

## Patentansprüche

1. Extraktionsverfahren von NN' Dibenzylthioharnstoff aus den Wurzeln einer Pflanze der Familie der Pendadiplandra Brazzeana, Varietät Brazzeana, charakterisiert durch die folgenden Etapen:
1) Ein Kg zerstampfter und getrockneter Wurzeln in einen 2 Liter Flacon gelber Farbe.
2) Dazu 1195% Äthylalkohol, anschließend die Mischung 4 Stunden bewegen.
3) Die aus 2) erhaltene Mischung 1 Stunde ruhen lassen.
4) Filtern und die gefilterte Mischung auffangen.
5) Distillation im Vakuum bei ungefähr 15 mm HG auf 40° bis zum Erhalt einer reinen konzentrierten hydroalkoholischen Lösung, indem man vermeidet einen trockenen Rest zu erhalten.
6) Hinzufügung von 250ml Tetrachlorkohlenstoff zu der in Etappe 5) erhaltenen hydroalkoholischen Lösung.
7) Eine Stunde lang bewegen.
8) Abstich und Sammlung der hydroalkoholischen finalen Phase, die den unlöslichen NN' Dibenzylthioharnstoff im Tetrachlorkohlenstoff enthält.

2. Verfahren entsprechend der Geltendmachung 1, dadurch charakterisiert, dass das Verfahren ohne Präzipitation des Produkts und ohne vollständige Verdampfung des Lösungsmittels verläuft, um den Kontakt mit dem Sauerstoff der Luft zu vermeiden.

3. Verfahren entsprechend der Geltendmachung 1, dadurch charakterisiert, dass die hydroalkoholische Phase den Dibenzylthioharnstoff als einzige aus der Wurzel der Pendadiplandra Brazzeana, Varietät Brazzena, extrahierte Zusammensetzung enthält und unlöslich ist im Tetrachlorkohlenstoff.

4. Verfahren entsprechend der Geltendmachungen 1, 2, 3, charakterisiert dadurch, dass der durch dieses Verfahren erhaltene Dibenzylthioharnstoff ein biologisches Antioxydant pflanzlichen Ursprungs ist.

5. Verfahren entsprechend der Geltendmachungen 1, 2, 3 et 4, charakterisiert dadurch, dass die H der Gruppierung -NH-, der der aktive Sitz der Zusammensetzung ist, erhalten bleiben.
